(19) Europäisches Patentamt
European Patent Office
Office européen des brevets

(11) Veröffentlichungsnummer: **0 008 387**
**B1**

(12) # EUROPÄISCHE PATENTSCHRIFT

(45) Veröffentlichungstag der Patentschrift: **25.11.81**

(51) Int. Cl.³: **A 47 J 36/38,** F 24 C 15/20, A 61 L 9/00

(21) Anmeldenummer: **79102689.1**

(22) Anmeldetag: **28.07.79**

(54) **Dunstabzugshaube zur Anordnung über Küchenherden.**

(30) Priorität: **12.08.78 DE 2835397**

(43) Veröffentlichungstag der Anmeldung: **05.03.80 Patentblatt 80/5**

(45) Bekanntmachung des Hinweises auf die Patenterteilung: **25.11.81 Patentblatt 81/47**

(84) Benannte Vertragsstaaten: **FR GB IT NL**

(56) Entgegenhaltungen:
**DE-A-1 909 861**
**DE-A1-2 511 825**
**DE-A1-2 539 743**
**DE-A1-2 654 170**
**US-A-4 188 226**

(73) Patentinhaber: **NEFF-WERKE Carl Neff GmbH, Bahnhof-Strasse 9-11, D-7518 Bretten (DE)**

(72) Erfinder: **Wachenfeld, Adolf, Kirchberg 24, D-7519 Oberderdingen (DE)**
Erfinder: **Witt, Horst, Joss Fritz-Strasse 48, D-7520 Bruchsal 4 (DE)**

(74) Vertreter: **Vogl, Leo, Dipl.-Ing., AEG-TELEFUNKEN Konsumgüter AG Abteilung Patente Muggenhofer Strasse 135, D-8500 Nürnberg (DE)**

EP 0 008 387 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Dunstabzugshaube zur Anordnung über Küchenherden

Die Erfindung betrifft eine Dunstabzugshaube zur Anordnung über Küchenherden mit einem die anfallenden Kochwrasen oder dgl. absaugenden und fördernden Gebläse, sowie mit einem vor der Ansaugseite des Gebläses angeordneten Fett- und Geruchsfilter.

Eine Dunstabzugshaube dieser Art ist aus der DE-A-2 654 170 bekannt. Die Fett- und Geruchsfilter bestehen bei einer solchen Dunstabzugshaube in der Regel aus Vlies- oder Filzmatten. Es hat sich nun gezeigt, daß sich innerhalb der Dunstabzugshaube infolge des Vorhandenseins warmer und feuchter Luft im Laufe der Zeit Pilz- und Bakterienkulturen bilden können, die sich besonders gerne im Vlies oder der Filzmatte von Fett- und Geruchsfiltern ansiedeln. Infolge der in den Fett- und Geruchsfiltern sich ablagernden Verunreinigungen aus den durchgeschleusten Kochwrasen und Kochdünsten ergibt sich dort für Pilz- und Bakterienkulturen ein idealer Nährboden. Die in den Fett- und Geruchsfiltern vorhandenen Pilze und Bakterien können durch Berührung, und insbesondere bei sogenannten Umlufthauben infolge der Luftumwälzung, gesundheitsschädlich für den Menschen sein. So ist es z. B. bekannt, daß durch Berührung und Übertragung dieser Pilze und Bakterien Mykosen und Infektionskrankheiten entstehen können. Ebenfalls nicht auszuschließen ist, daß solche Pilze und Bakterien durch die Luftumwälzung auch auf in den Küchenräumen abgestellten Nahrungsmittel gelangen.

Hier will die Erfindung nun Abhilfe schaffen. Die Erfindung, wie sie in den Ansprüchen gekennzeichnet ist, löst die Aufgabe, die Filtereinrichtung bei einer Dunstabzugshaube der eingangs genannten Art mit gesundheitlich unbedenklichen Mitteln zuverlässig keimfrei auszurüsten, um nicht nur die Entstehung bzw. Bildung von Bakterien sondern auch von Mykosen im Luftförderbereich der Dunstabzugshaube zu verhindern.

Durch die vorgeschlagene antibakterielle und antimykotische Ausrüstung der Fett- und Geruchsfilter wird die Entwicklung von Pilzen und Bakterien bzw. Mikroben innerhalb der Dunstabzugshaube von vornherein mit Sicherheit verhindert und damit ein Maximum an Keimfreiheit in der Küche und am Küchenarbeitsplatz erreicht.

An sich ist es in der Krankentherapie bekannt, bewegliche Filtergeräte zur Asepsis von Patienten zu verwenden, bei denen das Filtermaterial des Schwebstoff-Filters bakterizid ausgerüstet ist (DE-A-2 539 743). Eine solche Ausrüstung allein schützt aber Filtereinrichtungen nicht vor einem Pilzbefall.

Ein Ausführungsbeispiel der Erfindung ist in der Zeichnung dargestellt und wird an Hand dieser nachfolgend näher beschrieben. Die Zeichnung zeigt eine Dunstabzugshaube im Schnitt.

Im Gehäuse 1 einer Dunstabzugshaube ist ein die anfallenden Kochwrasen und dgl. absaugendes und förderndes Gebläse 2 untergebracht, dessen Ansaugöffnung 3 ein übliches Geruchsfilter 4 vorgeschaltet ist. Bei dem dargestellten Ausführungsbeispiel ist der Geruchsfilter 4 zwischen je einer Vlies- oder Filzmatte 5, 5' gebettet.

Vor dem Geruchsfilter 3 ist mit Abstand ein die gesamte Dunstabzugshauben-Ansaugfläche abdeckender Fettfilter 6 angeordnet, der ebenfalls aus einer Vlies- oder Filzmatte gebildet ist. Vor dem Fettfilter 6 ist eine senkrechte verlaufende Schließ- und Wrasenauffangklappe 7 angebracht. An der Oberseite des Gehäuses 1 befindet sich eine Luftaustrittsöffnung 8. Der erforderliche Druck- und Saugraum der Dunstabzugshaube ist mit 9 bzw. 10 bezeichnet.

Um nun sowohl im Inneren der Dunstabzugshaube als auch im Fettfilter 6 und in den die Geruchsfilter 4 beidseitig abdeckenden Vlies- oder Filzmatten 5, 5' die Bildung von Pilz- und Bakterienkulturen zu verhindern, sind der Fettfilter 6 und die Vlies- oder Filzmatten 5, 5' zweckmäßig mit antimykotischen und antibakteriellen Schutzmitteln ausgerüstet bzw. imprägniert. Bei Verwendung eines Geruchsfilters 4 ohne beidseitige Abdeckung durch eine Vlies- oder Filzmatte ist der Geruchsfilter selbst mit einem solchen Mittel ausgerüstet.

Als Imprägnierungs- oder Konservierungsmittel sind alle organischen und anorganischen Verbindungen einsetzbar, die eine zumindest entwicklungshemmende Wirkung auf Pilze und Bakterien aufweisen. Bevorzugt werden Konservierungsmittel mit möglichst breiter fungizider und bakteriozider Wirkung, die jedoch bezüglich der Toxizität unbedenklich sind. Als entwicklungshemmende Mittel sind u. a. verwendbar:

Organozinnverbindungen

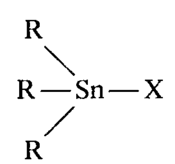

quaternäre Ammoniumsalze

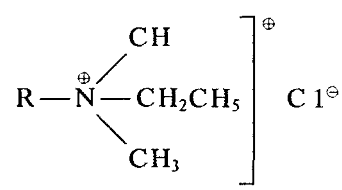

Fluor-Verbindungen, z. B. Natrium-fluorid oder Natrium-fluorsilikat, Organozinkverbindungen, z. B. Zinknaphtenat, Zinkdimethyldithiocarbamat, kupferhaltige Verbindungen, wie Kupfersulfat, Kupferchlorid, Kupferchromat, Kupferoleat, Kupferstearat, Kupfernaphtenat, sowie weitere kupferorganiche Verbindungen, weiterhin Guanidine, wie Dodecylguanidinchlorid, Chlorhexidin Phenol-Derivate, wie

Diphenylalkane, Dithiocarbamate

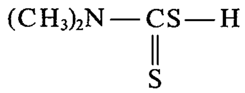

Naphthenate, wie Kupferhydroxynaphthenat.

Die genannten Imprägnierungsmittel können auf den Filtern bzw. Matten durch Tränken oder Aufwalzen sowohl während der Herstellung der Filter oder Matten als auch danach ein- bzw. aufgebracht werden. Die Imprägnierungsmittel werden dabei zweckmäßig in einer Größenordnung von etwa 0,1% bis 15%, bezogen auf das Gewicht der Filter bzw. Matten, in diesen eingelagert.

Das die genannten Imprägnierungsmittel bis etwa 130°C temperaturbeständig sind, erscheinen sie ohne Einschränkung für den vorgesehenen Zweck bestens geeignet. Auch bleibt die Wirksamkeit der Imprägnierungsmittel selbst nach mehrmaligem Waschen des Fettfilters erhalten. Die physikalisch-mechanischen Eigenschaften der Filter werden durch die antimikrobielle Ausrüstung ebenfalls in keiner Weise beeinträchtigt.

## Patentansprüche

1. Dunstabzugshaube zur Anordnung über Küchenherden mit einem die anfallenden Kochwrasen oder dergleichen absaugenden und fördernden Gebläse (2), sowie mit einem vor der Ansaugseite des Gebläses angeordneten Fett- und Geruchsfilter (6, 4), dadurch gekennzeichnet, daß die Fett- und Geruchsfilter (6, 4) neben einer bekannten antibakteriellen Ausrüstung zusätzlich mit einem antimykotischen Mittel ausgerüstet bzw. imprägniert sind, wobei als Ausrüstungs- bzw. Imprägnierungsmittel chemische Fungizide und Bakterizide dienen, die in einer Menge von etwa 0,1% bis 15% des jeweiligen Gewichtes der betreffenden Fett- und Geruchsfilter diesen zugesetzt werden.

2. Dunstabzugshaube nach Anspruch 1, mit einem beidseitig mit einer Vlies- oder Filzmatte (5, 5') abgedeckten Geruchsfilter, dadurch gekennzeichnet, daß die Ausrüstungs- bzw. Imprägnierungsmittel den Vlies- oder Filzmatten (5, 5') zugesetzt sind.

3. Dunstabzugshaube nach Anspruch 1 oder 2, dadurch gekennzeichnet, daß die Imprägnierungsmittel durch Tränken, Aufsprühen, Aufwalzen während oder nach der Herstellung der Fett- und Geruchsfilter (6, 4) bzw. Vlies- oder Filzmatten (5, 5') ein- bzw. aufgebracht werden.

4. Dunstabzugshaube nach Anspruch 1, dadurch gekennzeichnet, daß als Imprägnierungsmittel organische Zinnverbindungen, quaternäre Ammoniumverbindungen, Benzalkonium, Pentachlor-phenol-Fettsäureester und gegebenenfalls auch chlorierte Phenole verwendet werden.

5. Dunstabzugshaube nach Anspruch 1, dadurch gekennzeichnet, daß als Imprägnierungsmittel ein Compound aus hochwirksamen Fettsäureamiden, organischen Zinnverbindungen und quaternären Ammoniumsalzen unter Zusatz von Benzalkonium bzw. Benzalkoniumchlorid sowie geringen Mengen chlorierter Phenole dient.

## Claims

1. Fume removal hood for arrangement over kitchen ranges with a blower (2) sucking away and conveying the arising cooking vapours or the like as well as with a fat and smell filter (6, 4) arranged in front of the suction side of the blower, chracterised thereby, that the fat and smell filters (6, 4) are apart from a known antibacterial equipment additionally equipped or impregnated with an antimycotic agent, wherein chemical fungicedes and bactericides, which are added in a quantity of about 0.1% to 15% of the respective weight of the concerned fat and smell filter to these, serve as equipping or impregnating means.

2. Fume removal hood according to claim 1 with a smell filter covered at both sides by a fleece or felt mat (5, 5'), characterised thereby, that the equipping or impregnating means are added to the fleece or felt mats (5, 5').

3. Fume removal hood according to claim 1 or 2, characterised thereby, that the impregnating means are introduced or applied by soaking, spraying-on or rolling-on during or after the manufacture of the fat ans smell filters (6, 4) or fleece or felt mats (5, 5').

4. Fume removal hood according to claim 1, characterised thereby, that organic tin compounds, quaternary ammonium compounds, benzalkonium, pentachlor-phenol fatty acid esters and in a given case also chlorinated phenols are used as impregnating means.

5. Fume removal hood according to claim 1, characterised thereby, that a compound of highly active fatty acid amides, organic tin compunds and quaternary ammonium salts with the addition of benzalkonium or benzalkonium chloride as well as small quantities of chlorinated phenols serves as impregnating means.

## Revendications

1. Hotte filtrante destinée à être placée audessus de cuisinières et qui comporte une soufflerie (2) aspirant et évacuant la buée produite lors de la cuisson ainsi que des filtres (6, 4) disposés sur le côté d'aspiration de la soufflerie pour retenir les matières grasses et neutraliser les odeurs, caractérisée en ce que les filtres (6, 4) retenant les matières grasses et

neutralisant les odeurs sont imprégnés ou contiennent en plus d'un équipement antimicrobien habituel un produit mycostatique des agents fongicides et bactéricides chimiques additionnés dans une quantité comprise entre 0,1% et 15% en poids par rapport à celui des filtres retenant les matières grasses et neutralisant les odeurs, étant utilisés en tant que produit additionnel et d'imprégnation.

2. Hotte filtrante suivant la revendication 1 comportant un filtre désodorisant recouvert sur ses deux faces par une couche en voile de carde ou en feutre (5, 5'), caractérisée en ce que les agents supplémentaires ou d'imprégnation sont additionnés aux couches en voile de carde ou en feutre (5, 5').

3. Hotte filtrante suivant l'une des revendications 1 ou 2, caractérisée en ce que les agents d'imprégnation sont additionnés par inhibition, pulvérisation ou par laminage sur le filtre à graisse et le filtre désodorisant (6, 4) ou bien sur les couches en voile de carde ou en feutre (5, 5') pendant ou après la fabrication de ces filtres.

4. Hotte filtrante suivant la revendication 1, caractérisée en ce qu'on utilise en tant qu'agents d'imprégnation des composés organostamiques des composés d'ammonium quaternaire, de benzalconium, de l'ester pentachlorophénolique d'acide gras et le cas échéant étalement des phénols chlorés.

5. Hotte filtrante suivant la revendication 1, caractérisée en ce qu'on utilise en tant qu'agent d'imprégnation un mélange composé d'amides d'acide gras à activité élevée, de composés organo-stamiques et de sels d'ammonium quaternaire additionné de benzalconium ou de chlorure de benzalconium ainsi que de faibles quantités de phénols chlorés.

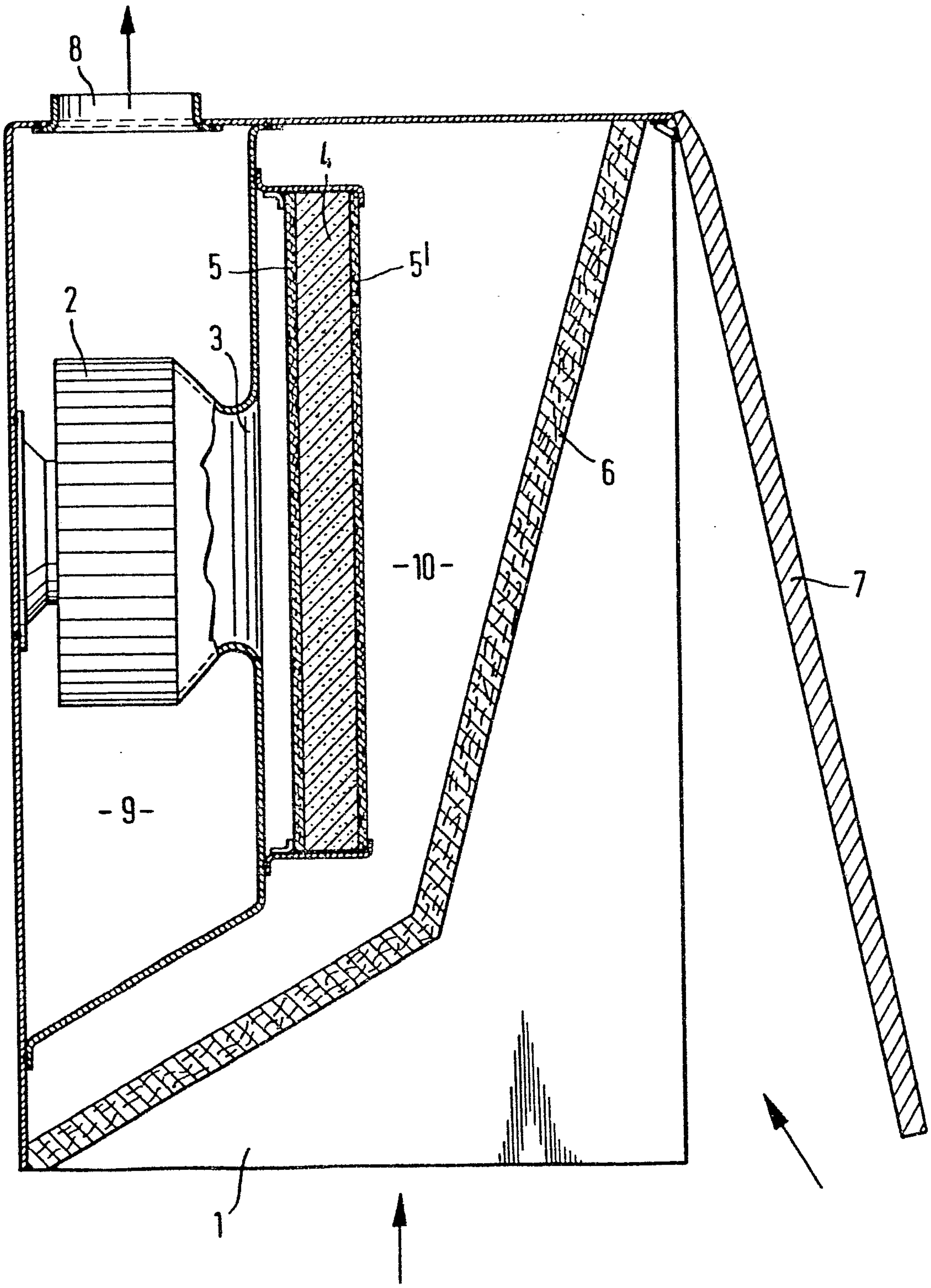
8
4
5
5'
2
3
6
-10-
7
-9-
1